# EUROPEAN PATENT SPECIFICATION

(11) **EP 1 514 860 B1**
(45) Date of publication and mention of the grant of the patent: **03.08.2011**
(21) Application number: 04077488.7
(22) Date of filing: 08.09.2004
(51) Int. Cl.: C07C 2/32

(54) **Catalytic composition for the oligomerization of ethylene comprising monocyclopentadienyl compounds of titanium**
Katalytische Zusammensetzung zur Oligomerisierung von Ethylen enthaltend Monocyclopentadienyltitan-Verbindungen
Composition catalytique pour l'oligomèrisation de l'ethylène comprenant des composés monocyclopentadienyl de titanium

(30) Priority: 11.09.2003 IT MI20031738
(43) Date of publication of application: 16.03.2005
(73) Proprietor: Polimeri Europa S.p.A., 20097 San Donato Milanese (MI) (IT)
(72) Inventor: Biagini, Paolo, 28069 Trecate Novara (IT); Bonsignore, Stefanio, 28100 Novara (IT); Santi, Roberto, (IT); Fusco, Roberto, 28100 Novara (IT)
(74) Representative: Coletti, Raimondo

(56) References cited:
- WO-A-02/066405
- US-A- 5 087 788
- JOURNAL OF POLYMER SCIENCE, POLYMER CHEMISTRY EDITION., vol. 38, 2000, pages 4258-4263, XP002286025 US JOHN WILEY AND SONS. NEW YORK.
- ANGEWANDTE CHEMIE. INTERNATIONAL EDITION., vol. 40, no. 13, 2001, pages 2516-2519, XP002286030 DEVCH VERLAG, WEINHEIM.

## Description

The present invention refers to a catalytic composition comprising titanium complexes, which can be used in selective oligomerization processes of ethylene to 1-hexene.

Linear α-olefins represent an important petrochemical material. Their applications, depending on the number of carbon atoms, extend from their use as comonomers in the production of polyethylenes, to their use as synthetic plasticizers and lubricants, and as intermediates in the production of detergent alcohols. In particular, linear α-olefins having from 4 to 8 carbon atoms are widely used as comonomers for obtaining polyethylenes with different degrees of density and crystallinity, particularly suitable for producing manufactured products by means of filming and injection moulding processes. Among these products, 1-hexene is certainly the most widely used.

The possible oligomerization of ethylene to 1-hexene, 1-octene and also 1-butene, appears to be an interesting synthesis technique as the request for these monomers is extremely high, but the possibility of obtaining the selective trimerization of ethylene to 1-hexene would be even more useful, thus combining the request for producing the most widely used monomer with the separation facility of the product from the reaction mix.

In accordance with US-A-3,644,563 (Shell), homogeneous catalysts are used for the oligomerization of ethylene, based on organometallic complexes of nickel, comprising a bidentate bond (P-O) on which the catalytic activity and selectivity depend. The catalytic precursor is prepared at 40°C by the reaction of NiCl₂ with said bidentate bond P-O (such as, for example, di-phenyl phosphino acetic acid and di-phenyl phosphino benzoic acid) in the presence of ethylene and a reducing agent, such as NaBH₄. Oligomerization is carried out at 120°C and 14 MPa. The olefins obtained according to this process have a high linearity and their molecular weights follow a Shulz-Flory distribution.

This process consequently has the disadvantage of requiring drastic conditions of pressure and temperature and giving a large distribution of α-olefins.

US-A-4,783,573 (Idemitsu) describes a process wherein ethylene is oligomerized at 3.5 MPa and 120°C, in the presence of a catalytic system which comprises ZrCl₄, aluminum alkyls and a Lewis base which can be selected from different classes of organic compounds containing hetero-atoms, such as alkyl disulfides, thioethers, thiophenes, phosphines and primary amines. C₄-C₈ olefins are mainly obtained, but considerable quantities of heavy olefins are also present and, in addition, their preparation requires, in any case, high temperature and pressure.

International patent application WO 92/10446 (IFP) describes a process for converting ethylene to linear α-olefins in the presence of a catalyst consisting of a zirconium alcoholate, an aluminum chloro-alkyl and an ether. Even if the catalyst is active under relatively bland conditions, neither is the selectivity of the process towards olefins having 4, 6 and 8 carbon atoms, nor the distribution of the product among these compounds, satisfactory, as the production of 1-butene is too consistent.

EP-A 681,106 (Phillips) describes catalytic systems based on chromium (III) alkanoates, which are generally activated with aluminum alkyl AlEt₃ in a mix with AlClEt₂, in the presence of a pyrrole or one of its alkaline salts, and a halogenating agent, preferably GeCl₄, used at temperatures around 100°C, with ethylene pressures higher than 40 atm. These chromium catalytic systems produce 1-hexene with a selectivity higher than 99% and a high activity only with a high ethylene pressure, as polyethylene is obtained at low pressures.

WO 02/066404, WO 02/066405, as also Angew. Chem. Int. Ed., volume 40 (nr.13) pages 2516-2519 (2001), Organometallics, volume 21 pages 5122-5135 (2002) and the 1st Blue Sky Conference on Catalytic Olefin Polymerization, Sorrento, June 17-20, 2002 (Poster nr.32), describe the use of particular titanium(IV) monocyclopentadienyl compounds, which, preferably activated with MAO, are capable of inducing the selective oligomerization of ethylene at temperatures of between 30 and 80°C and pressures of 0.2 - 1.0 MPa. According to the authors, the high selectivity in forming 1-hexene (typically from 80 to 95% with respect to the converted ethylene) is only obtained when an aryl substituent is linked, by means of a suitable group, to the cyclopentadienyl ligand present in the precursor of titanium(IV).

Finally, the publications "Macromolecules", volume 32 (1999) page 4491" and "Journal of Polymer Science" Part A: Polymer Chemistry, volume 38 (2000), pages 4258-4263, describe monocyclopentadienyl titanium complexes of the type Cp*TiX₃ [X=Me, OBz], activated with MAO or with suitable boron derivatives, which are capable of polymerizing ethylene, producing ethylene/hexene and/or ethylene/butene copolymers. The authors assume that the catalytic system is also capable of inducing the oligomerization of ethylene to 1-butene and/or 1-hexene, and of co-polymerizing the two olefins produced with further ethylene, thus forming the relative copolymers. Said olefins are not identified however, under the conditions described by the authors, and the copolymer proves to be the only product of the above-mentioned reactions.

The necessity of widening the range of processes and catalysts suitable for the oligomerization of ethylene to 1-hexene is consequently still strongly felt, possibly using low cost materials and reagents easily available on the market and.

The Applicant has now surprisingly found that it is possible to selectively produce 1-hexene by the trimerization of ethylene catalyzed by catalytic compositions containing mono-metallocene compounds of titanium, not necessarily substituted with benzyl groups. It is therefore possible to produce 1-hexene from ethylene by means of a wide range of titanium complexes, some of them available on the market, without having to resort to the complex preparation of the above-mentioned benzyl complexes.

A first object of the present invention therefore relates to a catalytic composition for the selective trimerization of ethylene to 1-hexene, comprising the reaction product of:
a) a monocyclopentadienyl compound of titanium having the following formula (I)

   [ATiRₙ]ₘ (I)

   wherein: Ti is a titanium atom in an oxidation state of +3 or, preferably, +4;
   A is an anion containing a η⁵-cyclopentadienyl ring coordinated to the titanium atom; each R independently represents a suitable group linked to the titanium atom through a covalent bond, selected from hydride, halide, a C₁-C₈ alkyl group, a C₃-C₁₂ alkyl silyl group, a C₅-C₈ cyclo alkyl group, a C₆-C₁₀ aryl group, a C₁-C₈ alkoxyl group, a C₁-C₃₀ carboxyl group, a C₂-C₁₀ dialkyl amide group;
   "n" is equal to 2 or 3 when the titanium is in oxidation state +3 or +4, respectively;
   "m" is an integer ranging from 1 to 4;
b) a non-monomeric oxyhydrocarbyl compound of aluminum, and
c) an organometallic hydrocarbyl compound selected from the hydrocarbyl compounds having the following formula (III):

   (R')ₘ M Xₛ - ₘ (III)

   wherein M is
   Al,
   R' is a hydrocarbyl group having from 1 to 15, preferably from 1 to 8, carbon atoms,
   X is chlorine or bromine,
   m is an integer ranging from 1 to s, preferably s, s is the valence of the metal Al and has the values of 3, according to the chemical nature of Al. characterized in that it is obtained by means of a process comprising the following steps in succession:
   i) putting said titanium complex (a) in contact and reacting, in a suitable inert liquid medium, with a quantity of said oxyhydrocarbyl compound of aluminum (b), so that the atomic ratio Al/Ti ranges from 50 to 2000, preferably from 300 to 1000, and
   ii) putting the product of the previous step (i) in contact and reacting with said organometallic hydrocarbyl compound (c) in such a quantity that the atomic ratio M/Ti ranges from 20 to 200 and preferably so that the ratio Al/M between the aluminum in said oxyhydrocarbyl compound (b) and the metal M in (c) ranges from 1 to 20.

A further object of the present invention relates to a process for the oligomerization of ethylene to prevalently form 1-hexene, comprising reacting ethylene under oligomerization conditions, in the presence of the above catalytic composition.

Further objects of the present invention will appear evident in the following description and claims.

Component (a) of the catalytic composition according to the present invention, can consist of any suitable titanium monocyclopentadienyl compound having an oxidation state of +3 or +4. Ti compounds having an oxidation state of +4 are preferred as they are more easily available and require fewer precautions for their preservation.

For the purposes of the present invention, it not necessary for said titanium compound (a) to be soluble in the inert organic liquids commonly used, as will seen further on, in the preparation of the catalytic composition, as it can also be used in solid form or as a suitable suspension. Monocyclopentadienyl compounds of Ti soluble in suitable organic solvents are preferred however.

In the metallocene complexes having formula (I) which form component (a) in the catalyst suitable for the present invention, group A is an anion containing an η⁵-cyclopentadienyl ring, preferably deriving (formally by the extraction of a H⁺ ion) from a molecule of cyclopentadiene, indene or fluorene, or from a homologous product of the above compounds, wherein one or more carbon atoms of the molecular skeleton, (included or not included in the cyclopentadienyl ring) are substituted by C₁-C₈ alkyl or silyl alkyl groups, or by C₆-C₁₈ aryl or aryloxy groups, or by C₁-C₈ alkoxyl groups. Said group A can also be condensed with one or more other aromatic rings, as in the case, for example, of 4,5-benzoindenyl. Typical A groups are, for example, cyclopentadienyl (C₅H₅), indenyl, 4,5,6,7-tetrahydroindenyl. A groups wherein said η⁵-cyclopentadienyl ring includes an aryl methyl substituents (for example benzyl), possibly substituted, are preferably excluded.

According to the present invention, each R group in formula (I) can independently represent a hydride, a halide, such as chloride or bromide, a C₁-C₈ alkyl group, such as, for example, methyl, ethyl, butyl, isopropyl, isoamyl, octyl, benzyl, a C₃-C₁₂ alkyl silyl group, such as, for example, trimethyl silyl, triethyl silyl, tributyl silyl, a cyclo alkyl group such as cyclopentyl or cyclohexyl, a C₆-C₁₀ aryl group such as phenyl or tolyl, a C₁-C₈ alkoxy group, such as, for example, methoxy, ethoxy, iso- or sec-butoxy, a C₁-C₃₀, preferably C₁-C₁₅, carboxylic group, or again a C₂-C₁₀ dialkyl amide group, an oxide, a hydroxide, a sulfide, a C₁-C₁₂ alkyl sulfide group, such as, for example, methyl sulfide, n-butyl sulfide, phenyl sulfide.

Typical examples of R groups suitable for the purposes of the present invention are: fluoride, chloride, bromide, iodide, dimethyl amide, dibutyl amide, diphenyl amide, bis(trimethyl silyl) amide, dimethyl phosphide, diphenyl phosphide, methoxide, ethoxide, iso-propoxide, butoxide, tert-butoxide, phenoxide, 2,6-di-tert-butyl phenoxide, p-fluoro phenoxide, pentafluoro phenoxide, acetate, propionate, 2-ethyl hexanoate, versatate, naphthenate, benzoate, N,N-diethyl-carbamate, N,N-dibutyl-carbamate, N,N-di-isopropyl-carbamate, N,N-dicyclo-hexyl-carbamate, N,N-diphenyl-carbamate, hydride, methyl, tert-butyl, neopentyl, phenyl, benzyl, p-fluoro-phenyl, pentafluoro-phenyl. Chloride, C₁-C₈ alkoxide and C₂-C₁₂ carboxylate ligands are particularly preferred as the compounds formed therefrom can be easily found on the market and due to their solubility in the solvents commonly used in the preparation of the above complexes and in the processes catalyzed thereby.

Metallocenes having the previous formula (I) are generally known in the art and can be prepared by means of one of the usual methods suitable for the purpose, described in synthesis handbooks of organometallic compounds, or in the vast patent literature relating to the use of metallocenes in the polymerization of olefins. For illustrative purposes, reference is made to the patent publications EP-A 416,815, EP-A 528,287, EP-A 574,794, EP-A 576,970, EP-A 577,581, WO 86/05788, US 5,264,405 and US 5,304,523.

Non-limiting examples of complexes having formula (I), according to the present invention, are listed herebelow:
(η⁵-C₅H₅)Ti(OCOMe)₃; (η⁵-C₅H₅)Ti (OCOPh)₂; (η⁵-C₅H₅) TiMe₃ ;
(η⁵-C₅H₅)TiCl₃; (η⁵-C₅H₅) TiBz(Cl)₂;(η⁵-C₅H₅) TiBz₃;
(η⁵-C₅H₅)TiBr₃; (η⁵-Ind)TiCl₃; (η⁵-C₅H₅)Ti (OPh)₃;
(η⁵-C₅H₅)TiCl(OCOPH)₂; (η⁵-Ind) Ti (OEt)₃;(η⁵-C₅H₅) Ti (OCOCF₃)₂;
(η⁵-C₅H₅)Ti(OMe)₃; (η⁵-C₅H₅)Ti (OCOMe)₂; (η⁵-C₅H₅) TiCl₂;
(η⁵-C₅H₅)Ti[OCO (CH₂)ₙMe]₃; (η⁵-Ind)TiBz₃; (η⁵-Ind) Ti (OMe)₃;
(η⁵-Ind)TiMe₃; (η⁵-Ind)Ti(OCOMe)₃; (η⁵-Ind)Ti(OPh)₃;
(η⁵-THInd)TiCl₃; (η⁵-THInd) TiBr₃; (η⁵-THInd) Ti (OCOCF₃)₃;
(η⁵-THInd)TiMe₃; (η⁵-THInd) Ti (OMe)₃;(η⁵-THInd)Ti(OBu)₃ ;
(η⁵-THInd)Ti[OCO (CH₂)ₙMe]₃; (η⁵-THInd) Ti (OCOPh)₃;
(η⁵-THInd) Ti (SMe)₃; (η⁵-Ind)Ti (OCONEt₂)₃;

In the above formulae the following abbreviations were used: Me = Methyl, Ind = indenyl, THInd = 4,5,6,7-tetrahydro-indenyl, Ph = phenyl, Bz = benzyl, Et = ethyl.

The subscript "n", in the formulae in which it appears, stands for an integer between 1 and 16.

Catalytic compositions wherein the above complex having formula (I) is carried on a solid inert medium consisting, for example, of a porous inorganic solid, such as silica, alumina, silico-aluminates, possibly dehydrated and activated according to the methods known in the art, or consisting of a polymeric organic solid such as polystyrene, are also included in the scope of the present invention.

The oxyhydrocarbyl compounds of aluminum, suitable as component (b) in the formulation of the present catalytic composition, are compounds wherein at least 50% of the aluminum, preferably at least 90%, is linked to at least one oxygen atom and to at least one organic group consisting of a linear or branched C₁-C₁₀ alkyl, preferably C₁-C₄, alkyl or a C₆-C₁₂ aryl.

According to the present invention, said oxyhydrocarbyl compound of aluminum is preferably an aluminoxane. It is well known that aluminoxanes are oligomeric compounds containing Al-O-Al bonds, with a varying O/Al ratio, which can be obtained, according to the known technique, by the reaction, under controlled conditions, of an aluminum alkyl, or aluminum alkyl halide, with water or other compounds containing controlled amounts of available water, as, for example, in the case of the reaction of aluminum trimethyl with a hydrated salt, such as aluminum sulphate hexahydrate, copper sulfate pentahydrate, and iron sulfate pentahydrate. Aluminoxanes preferably used for the formation of the polymerization catalyst of the present invention are cyclic and/or linear oligo- or poly-meric compounds, characterized by the presence of repetitive units having formula (II): 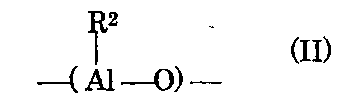 wherein R² is a C₁-C₄ alkyl group, preferably methyl.

Said aluminoxanes normally contain from 4 to 70 repetitive units per molecule, which may not be all the same, but contain different R² groups in the case of aluminoxanes with a mixed structure. Practical preparation examples of linear and/or cyclic aluminoxanes are provided, among others, in European patent applications EP-A 272, 584 and EP-A 421,659, and in the US patent 4,978,730, mentioned above.

Methylaluminoxane (MAO), which is preferably used according to the present invention, is a commercially available product, both pure and in solution in an inert hydrocarbon, normally toluene or heptane. Methylaluminoxane in a mixture with up to 10% by weight of aluminum trimethyl, which improves its stability with time, is also known and commercially available. It has been found however that the use of said MAO containing an aluminum alkyl is not equivalent to the combination of components (b) and (c), and is not capable of forming the present catalytic composition without the addition of a further quantity of alkyl metal (particularly aluminum alkyl), according to the method of the present invention

Component (c) of the present catalytic composition is selected from the hydrocarbyl compounds having the following formula (III):

(R')ₘM Xₛ-ₘ (III)

wherein M is
Al,
R' is a hydrocarbyl group having from 1 to 15, preferably from 1 to 8, carbon atoms,
X is chlorine or bromine,
m is an integer ranging from 1 to s, preferably s,
s is the valence of the metal Al and has the values of 3, according to the chemical nature of Al.

The above-mentioned organometallic compound in component (c) does not include the class of compounds defined as component (b).

Typical, non limiting examples of compounds having formula (III) are halides of aluminum alkyl, such as aluminum ethyl sesquichloride (Et₃Al₂Cl₃), dibutyl aluminum chloride, and aluminum alkyls, such as aluminum trimethyl, aluminum triethyl, aluminum tributyl, aluminum trihexyl, and mixtures of the above.

Aluminum alkyls are particularly preferred for the purposes of the present invention. These, in certain cases, are dimeric or oligomeric, such as, for example, the above-mentioned aluminum ethyl sesquichloride.

The catalytic composition according to the present invention can be prepared with a two-step method including, in step (i), the contact of components (a) and (b) as defined above, within a wide range of pressures and temperatures, preferably in the presence of an inert liquid medium. This can be effected either outside the utilization environment, i.e. outside the oligomerization reactor (preformed catalyst) or "in situ", inside the reactor, preferably in the same inert liquid medium used for the oligomerization of ethylene. The contact between the two components is normally effected by mixing the same in said liquid medium, preferably previously preparing the components already in solution. In the mixture of step (i), component (a) preferably has a concentration of between 3 and 10 mmoles/liter and component (b) an Al concentration of between 0.15 and 20.0 moles/liter.

The pressures conveniently used range from atmospheric pressure, preferable for preparing the preformed catalyst, to the pressure used in the subsequent ethylene oligomerization, i.e. up to about 7 MPa, in the case of preparation "in situ". The preparation temperature of the catalytic composition preferably ranges from -30 to 80°C.

The order in which components (a) and (b) are added, is not particularly important. In the case of the preformed catalytic system, component (a), comprising the desired amount of compound having formula (I), is preferably added to a solution of the oxyhydrocarbyl compound (b), in a suitable inert solvent, both in pure form, in liquid or solid state, and diluted in a suitable inert solvent, preferably the same as component (b).

Said inert liquid medium is preferably selected from aromatic hydrocarbons, also partially halogenated, such as benzene, toluene, xylenes, mesitylene, chloro-benzene, fluoro-benzene, aliphatic hydrocarbons such as pentane, hexane, heptane, octane, alicyclic hydrocarbons such as cyclohexane, methyl cyclohexane, or any mixture of two or more of the same. Toluene is preferably used.

According to the present invention, components (a) and (b) can also independently include a solid inert material as carrier, preferably selected from organic and inorganic inert solids, generally used for this purpose in analogous oligomerization or polymerization processes of olefins, such as, for example, alumina, silica, silico-aluminas, titania, zirconia, polystyrene. This inert solid carrier, when used, preferably consists of 40 to 90% by weight of the catalytic composition, excluding the weight of the possible solvent. Methods for supporting the catalyst are known to the skilled person in the art, for example by deposition and adsorption. According to a particular aspect, in step (i) said carrier can also be contemporaneously in contact with the two components (a) and (b) during the reaction, or with their reaction product.

The two components (a) and (b) are put in contact with each other and mixed in such quantities that the atomic ratio Al/Ti ranges from 50 to 2000, preferably from 200 to 1000. The reaction is normally complete within a few minutes after the contact of the components, for example from 2 to 10 minutes, depending on the temperature of the mixture. Nevertheless, according to a particular aspect of the present invention, the reaction product thus obtained can be advantageously left to rest (aging) for a period of between 30 minutes up to 24 hours or more, for example about an hour at 60°C, or about 24 hours at 10°C (refrigerator temperature) before being put in contact with component (c).

The product obtained in preparation step (i), is then mixed and reacted in step (ii) with said component (c) in order to obtain the desired catalytic composition. In accordance with this, component (c) is advantageously used in such proportions that the atomic ratio between M in formula (III) and titanium in the metallocene complex, normally ranges from 50 to 5000, preferably from 200 to 2000. The activation of the mixture is extremely rapid and is normally ready for use a minute after mixing. There are no particular limitations with respect to the temperature at which the contact and reaction between the compounds should be effected in step (ii). This is preferably selected from -20 to 50°C, more preferably from 0 to 30°C, even more preferably a temperature of 20 - 25°C is selected.

According to a preferred aspect of the present invention, the product of step (i) is obtained separately, and then introduced into the oligomerization reactor containing the desired quantity of component (c), preferably aluminum trialkyl, for completing step (ii). According to this preferred method, the reactor may already contain ethylene at the moment of introduction of the mixture of (a) and (b), at a pressure which can also be that subsequently maintained during the oligomerization process, thus advantageously effecting the start of the desired reaction as soon as the catalytic composition has been formed.

The mixture thus obtained is catalytically active, and can be used immediately or left to age, without undergoing substantial modifications of its characteristics, for times varying from a few minutes to a week, preferably from 30 minutes to 4 hours, at a temperature ranging from 0 to 25°C.

The process for the oligomerization of ethylene, which represents a further object of the present invention, comprises putting the above-mentioned catalytic composition in contact, under oligomerization conditions, with ethylene, or a gas containing ethylene, preferably in the presence of a suitable solvent and/or diluent selected from aliphatic, aromatic, cyclo-aliphatic hydrocarbons for the time sufficient for forming the desired amount of 1-hexene. In the preferred embodiment, an aromatic solvent/diluent having from 6 to 12, more preferably from 7 to 9, carbon atoms, is used.

The contact time between ethylene and catalytic composition can be suitably selected by experts in the field, in order to obtain the desired amount of 1-hexene. Contact times preferably ranging from 30 to 90 minutes give an optimal conversion to 1-hexene, so as to minimize the formation of by-products. The dimensions of the reaction equipment are selected by the designer so as to allow the desired contact or reaction times, in both continuous and batch processes.

The gas containing ethylene which can be used in the process of the present invention, includes an inert gas containing ethylene, preferably in a concentration not lower than 50% vol./vol., polymerization grade ethylene (for example high purity ethylene). In the preferred embodiment, the process of the present invention uses high purity ethylene.

The temperature and pressure of the oligomerization process of the present invention, can be selected from those normally used in the art for analogous processes. In particular, the temperature advantageously ranges from 0 to 100°C, preferably from 10 and 50°C; pressure is normally lower than 10 MPa, preferably from 0.05 to 7 MPa, even more preferably from 0.2 to 4 MPa.

The contact time (essentially coinciding with the time of the oligomerization reaction), in relation to the temperature, pressure and concentration, according to measurements easily obtainable by empirical methods during the normal experimentation necessary for setting up the process, generally ranges from 10 minutes to 10 hours, preferably from 30 minutes to 120 minutes.

According to a typical non-limiting embodiment of the present invention, the catalytic composition and ethylene are charged into the reactor at the desired pressure, for example 3 MPa, and this pressure is kept constant during the oligomerization reaction.

The reaction products mainly consist of 1-hexene, essentially without 1-butene (traces). Lower quantities, normally ranging between 10 and 40% by weight with respect to the weight of ethylene used, of 1-octene and higher oligomers, up to the ethylene polymer itself, are also obtained. The amount of undesired higher oligomers, which represent an inevitable process by-product, is significantly reduced. The process carried out in the presence of the catalytic composition according to the present invention, under the preferred pressure and temperature conditions, i.e. P between 2 and 4 MPa, and T between 60 and 140°C, allows an oligomerization product consisting of over 70% weight of 1-hexene, to be obtained.

The α-olefins thus produced can be separated from the reaction raw product according to methods known to experts in the field, particularly by means of fractionated distillation.

The following examples are provided for a better understanding of the present invention, and should in no way be considered as representing a limitation of the overall scope of the claims.

### EXAMPLES

The analytical techniques and characterization methods listed below and briefly illustrated, were used in the following examples.

The quantitative analysis of the ethylene oligomerization products was effected by gas chromatography, using a "Fisons" mod. 9000 instrument equipped with a Hewlett-Packard capillary column "Pona" [50 m x 0.2 mm x 0.5 microns]. The calculation of the quantities of each α-olefin was effected using the coefficient obtained from calibrations effected with 1-butene, 1-hexene, 1-octene, 1-decene and 1-dodecene, respectively (all products available on the market with purities > 99%), in the presence of 1,3,5-trimethyl benzene (>99.8 - Fluka) as internal standard.

The characterization of the products present in the reaction mixtures was effected by gas-chromatography/mass spectrometry (GC-Mass) using a Finnigan TSQ 700 instrument.

The following commercial reagents were used during the preparations described in the examples:

| | |
|---|---|
| trimethyl aluminum (TMA) (AlMe₃) | ALDRICH |
| triethyl aluminum (TEA) (AlEt₃) | ALDRICH |
| diethyl aluminum chloride (DEAC) (AlEt₂Cl) | ALDRICH |
| ethyl aluminum dichloride (EADC) (AlEtCl₂) | ALDRICH |
| methylaluminoxane (MAO) (EUROCEN AL 5100/10T) | |
| (10% solution by weight, containing 6-8% | |
| by weight of methylaluminoxane and 2-4% by weight of | |
| trimethyl aluminum, respectively) | WITCO |
| cyclopentadienyl titanium trichloride CpTiCl₃ | STREM |
| indenyl titanium trichloride IndTiCl₃ | STREM |

The reagents and/or solvents used and not listed above, are those commonly adopted in laboratory techniques and on an industrial scale, and can be easily obtained from specialized sellers in the field.

### EXAMPLE 1: preparation of the catalytic composition.

0.011 mmoles (1.1 ml) of cyclopentadienyl titanium trichloride, as a 0.010 M solution in toluene, and 4 ml of a toluene solution of methylaluminoxane (5.8 mmoles of total Al) are introduced, in this order, into a suitable tailed 50 ml flask. A limpid solution is obtained, after brief stirring, to which 0.62 mmoles of trimethyl aluminum, as an approximately 0.1 M solution in toluene, are added, after a few minutes at room temperature. An olive-green solution is almost immediately formed, containing the desired catalytic composition, which is transferred to the oligomerization reactor, within a few minutes.

### EXAMPLE 2

The same procedure was conducted as described in the previous example 1, using the same quantities of reagents, but instead of adding trimethyl aluminum to the solution of the titanium and MAO complex, the latter is directly transferred to the oligomerization reactor containing trimethyl aluminum (0.72 mmoles, of which 0.62 mmoles are reagent and 0.10 mmoles added as scavenger), so as to obtain the formation of the catalytic composition in situ.

### EXAMPLE 3

The same procedure was conducted as described in the previous example 1, using the same quantities of reagents, with the only difference that the solution of catalytic composition is left to age by maintaining it for 60 minutes at 25°C before transferring it to the oligomerization reactor, following the usual methods.

### EXAMPLE 4

The same procedure was conducted as described in the previous example 2, with the only difference that 0.010 mmoles of indenyl titanium trichloride (2.0 ml as an 0.005 M solution in toluene) are used, instead of 0.011 mmoles of CpTiCl₃ and 1.34 mmoles of AlMe₃ instead of 0.72.

### EXAMPLE 5 (Comparative)

The same procedure was conducted as described in the previous example 2, but no quantity of AlMe₃ is introduced into the reactor, except for the usual 0.10 mmoles as scavenger.

### EXAMPLE 6 (Comparative)

0.022 mmoles (2.2 ml) of cyclopentadienyl titanium trichloride as an 0.010 M solution in toluene, are introduced into the reaction reactor, already prepared by means of the usual operations, together with ethylene (1 MPa) and AlMe₃ (1.34 mmoles). No amount of MAO is added. The oligomerization reaction is then effected.

### EXAMPLE 7 (Comparative)

0.011 mmoles (1.1 ml) of cyclopentadienyl titanium trichloride, as an 0.010 M solution in toluene, 0.62 mmoles of trimethyl aluminum, as an approximately 0.1 M solution in toluene, and 4 ml of a toluene solution of methylaluminoxane (EUROCEN AL 5100/10T) (5.8 mmoles of total Al) are introduced, in this order, into a 50 ml tailed flask, maintained under nitrogen. An olive-green limpid solution is obtained after brief stirring, which is transferred to the reaction reactor to test its catalytic activity. In this comparative example, the order of addition of components (b) and (c), according to the definition of the present invention, was inverted with respect to example 1.

### EXAMPLE 8 (Comparative)

The same procedure was conducted as described in the previous example 7, with the only difference that 0.010 mmoles of indenyl titanium trichloride (2.0 ml as an 0.005 M solution in toluene) are used instead of 0.011 mmoles of CpTiCl₃, and 1.34 mmoles of AlMe₃ instead of 0.72.

### EXAMPLES 9 to 12 (ethylene oligomerization according to the invention)

The following examples 9 to 12 refer to a series of ethylene oligomerization tests for the preparation of 1-hexene according to the present invention, carried out using catalytic compositions comprising one of the titanium complexes according to general formula (I). The specific conditions of each example and the results obtained are shown in Table 1 below, which indicates, in order, the example number, the reference example for the preparation of the catalytic composition, the relative amount of titanium used, the reaction temperature and pressure, the amount of 1-hexene obtained, expressed as grams of olefin per gram of titanium metal per hour (gₕₑₓₑₙₑ/(g_{Ti}·h), the amount of polymer produced, expressed as gram of polymer per gram of titanium metal per hour (g_{pol.}/(g_{Ti}·h) and the reaction selectivity, expressed as grams of 1-hexene produced per gram of converted ethylene, which corresponds to the total amount of oligo- and poly-merization products [gₕₑₓₑₙₑ/(gₒₗ + g_{pol.})].

The oligomerization is carried out in an 0.5 liter pressure reactor equipped with a stirrer and magnetic anchor and an external jacket connected to a heat exchanger for the temperature control. The reactor is previously flushed by maintaining it under vacuum (0.1 Pascal) at a temperature of 80°C for at least 2 hours.

180 g of anhydrous toluene (or other hydrocarbon solvent) and possibly an aluminum alkyl or alkyl chloride, are introduced into the reactor in such a quantity as to form a solution having a concentration of about 5·10⁻⁴ M, acting as scavenger. The reactor is then heated to the desired polymerization temperature and "polymerization grade" gaseous ethylene is fed, by means of a plunged pipe, until the desired total equilibrium pressure, as specified, for example, in Table 1 below, is reached. The reaction is carried out for 60 minutes, the reactor is then depressurized and 2 ml of methanol are added to deactivate the catalyst. The reaction mix is analyzed by gas-chromatography, in order to determine the types and quantities of oligomers obtained; the polymeric fraction, possibly formed, is subsequently coagulated by adding a large excess of methanol, the solid product is filtered, dried in a ventilated oven for 10 hours at 60°C and the weight of the polymer obtained is determined. The formation of C₈₋₁₀ olefins was also observed, in amounts varying from 0.5 to 3% with respect to the 1-hexene produced.

### EXAMPLES 13 to 17 (Comparative)

The same procedure was conducted as described in examples 8 to 11, using the catalytic compositions and reaction conditions specified in table 1 below.

**Table 1: Ethylene oligomerization. Each reaction had a duration of 1 hour.**

| Ex. | Ti | Cat.Comp. | AlMe₃ | T | P_{(C2H4)} | 1-butene | 1-hexene | Polymer | Select. (%) |
|---|---|---|---|---|---|---|---|---|---|
| | (mmoles) | Ex. Nr. | (mmoles) | (°C) | (MPa) | (g/g_{Ti}·h) | (gₕₑₓₑₙₑ/g_{Ti}·h) | (gₚₒₗ/g_{Ti}·h) | [gₕₑₓₑₙₑ/(gₒₗ + gₚₒₗ] |
| 9 | 0.011 | 2 | 0.62 | 35 | 1.0 | 0 | 1738 | 1138 | 60 |
| 10 | 0.011 | 3 | 1.50 | 35 | 1.0 | 0 | 2170 | 421 | 82 |
| 11 | 0.011 | 2 | 0.62 | 50 | 3.0 | 0 | 2440 | 4269 | 36 |
| 12 | 0.010 | 4 | 5.8 | 35 | 1.0 | n.d. | 651 | 1501 | 30 |
| 13(*) | 0.017 | 5 | --- | 35 | 1.0 | 0 | 212 | 2095 | 10 |
| 14(*) | 0.022 | 6 | 1.24 | 35 | 1.0 | 150 | 0 | traces | n.d. |
| 15(*) | 0.011 | 7 | 0.62 | 35 | 1.0 | 0 | 459 | 842 | 34 |
| 16(*) | 0.011 | 7 | 0.62 | 50 | 3.0 | 0 | 852 | 4801 | 16 |
| 17(*) | 0.010 | 8 | 5.8 | 35 | 1.0 | n.d. | 650 | 3285 | 16 |

| | | | | | | | | | |
|---|---|---|---|---|---|---|---|---|---|
| (*) Comparative example | | | | | | | | | |

## Claims

1. A catalytic composition which can be used in the selective trimerization of ethylene to 1-hexene, comprising the reaction product of:
a) a monocyclopentadienyl compound of titanium having the following formula (I):
[ATiRₙ]ₘ (I)
wherein: Ti is a titanium atom in an oxidation state of +3 or, preferably, +4;
A is an anion containing a η⁵-cyclopentadienyl ring coordinated to the titanium atom;
each R independently represents a group covalently σ-bonded to the titanium atom, selected from hydride, halide, a C₁-C₈ alkyl group, a C₃-C₁₂ alkyl silyl group, a C₅-C₈ cyclo alkyl group, a C₆-C₁₀ aryl group, a C₁-C₈ alkoxyl group, a C₁-C₃₀ carboxyl group, a C₂-C₁₀ dialkyl amide group;
[ATiRₙ]ₘ (I)
"n" is equal to 2 or 3 when the titanium is in oxidation state +3 or +4, respectively;
"m" is an integer ranging from 1 to 4;
b) a non-monomeric oxyhydrocarbyl compound of aluminum, and
c) an organometallic hydrocarbyl compound
selected from compounds having the following formula (III):
wherein M is
Al,
R' is a hydrocarbyl group having from 1 to 15 carbon atoms,
X is chlorine or bromine,
m is an integer ranging from 1 to s, preferably s,
s is the valence of the metal Al and has the value of 3, according to the chemical nature of Al. **characterized in that** it is obtained by means of a process comprising the following steps in succession:
i) putting said titanium complex (a) in contact and reacting, in a suitable inert liquid medium, with a quantity of said oxyhydrocarbyl compound of aluminum (b), so that the atomic ratio Al/Ti ranges from 50 to 2000, preferably from 300 to 1000, and
ii) putting the product of the previous step (i) in contact and reacting with said organometallic hydrocarbyl compound (c) in such a quantity that the atomic ratio Al/Ti ranges from 20 to 200.

2. The composition according to claim 1, wherein, in formula (I), said R is selected from hydride, halide, a C₁-C₈ alkyl group, a C₃-C₁₂ alkyl silyl group, a C₅-C₈ cyclo alkyl group, a C₆-C₁₀ aryl group, a C₁-C₈ alkoxyl group, a C₁-C₃₀ carboxyl group, a C₂-C₁₀ dialkyl amide group, and said index "m" is equal to 1.

3. The composition according to one of the claims 1 and 2, wherein the group A in formula (I) is different from an η⁵-cyclopentadienyl ring comprising an aryl methyl substituent.

4. The composition according to any of the previous claims, wherein said compound having formula (I) of component (a) is soluble in the liquid medium of step (i).

5. The composition according to any of the previous claims, wherein said oxyhydrocarbyl compound of aluminum in component (b) is a polymeric aluminoxane, preferably methylaluminoxane.

6. The composition according to any of the previous claims, wherein in said step (ii) the Al/Al ratio between the aluminum in said oxyhydrocarbyl compound (b) and aluminium in (c) ranges from 1 to 20.

7. The composition according to any of the previous claims, wherein said steps (i) and (ii) are carried out *in situ* in a reactor for the trimerization of ethylene.

8. The composition according to any of the previous claims, wherein said inert liquid medium is selected from aromatic hydrocarbons, preferably toluene.

9. The composition according to any of the previous claims, subjected to a period of aging after completing step (i).

10. The composition according to any of the previous claims, subjected to a period of aging after completing step (ii).

11. A process for the oligomerization of ethylene to prevalently produce 1-hexene, **characterized in that** ethylene, or a gas containing ethylene, is put in contact, under suitable conditions, with said catalytic composition according to any of the previous claims from 1 to 10.

12. The process according to claim 11, wherein said oligomerization of ethylene is carried out at a temperature ranging from 0°C to 100°C and a pressure lower than 10 MPa.

13. The process according to claim 12, wherein said oligomerization of ethylene is carried out at a temperature ranging from 10°C to 50°C and a pressure ranging from 0.05 to 7 MPa.

14. The process according to claim 12 or 13, wherein said oligomerization is carried out in the presence of a solvent and/or diluent.

15. The process according to claim 14, wherein said solvent/diluent is selected from aromatic hydrocarbons

16. The process according to any of the claims from 11 to 15, wherein said catalytic composition is preformed before contact with ethylene.

17. The process according to any of the claims from 11 to 15, wherein said catalytic composition is formed in *situ*, in the presence of ethylene.

18. The process according to any of the claims from 11 to 17, comprising at least one separation step of 1-hexene.

## Patentansprüche

1. Katalytische Zusammensetzung, welche in der selektiven Trimerisierung von Ethylen zu 1-Hexen verwendet werden kann, umfassend das Reaktionsprodukt von:
a) einer Monocyclopentadienyl-Verbindung von Titan mit der folgenden Formel (I):
[ATiRₙ]ₘ (I)
worin: Ti ein Titanatom in einer Oxidationsstufe von +3 oder vorzugsweise +4 ist;
A ein Anion ist, das einen η⁵-Cyclopentadienylring enthält, der mit dem Titanatom koordiniert ist;
R jeweils unabhängig eine Gruppe darstellt, die an das Titanatom kovalent σgebunden ist, ausgewählt aus Hydrid, Halid, einer C₁-C₈ Alkylgruppe, einer C₃-C₁₂ Alkylsilylgruppe, einer C₅-C₈ Cycloalkylgruppe, einer C₆-C₁₀ Arylgruppe, einer C₁-C₈ Alkoxylgruppe, einer C₁-C₃₀ Carboxylgruppe, einer C₂-C₁₀ Dialkylamidgruppe;
"n" gleich 2 oder 3 ist, wenn das Titan in einer Oxidationsstufe von +3 bzw. +4 vorliegt;
"m" eine ganze Zahl im Bereich von 1 bis 4 ist;
b) einer nichtmonomerischen Oxyhydrocarbyl-Verbindung von Aluminium, und
c) einer organometallischen Hydrocarbylverbindung, ausgewählt aus Verbindungen mit der folgenden Formel (III):
(R')ₘMXₛ₋ₘ (III)
worin M Al ist,
R' eine Hydrocarbylgruppe mit 1 bis 15 Kohlenstoffatomen ist,
X Chlor oder Brom ist,
m eine ganze Zahl im Bereich von 1 bis s ist, vorzugsweise s, wobei s die Wertigkeit des Metalls Al ist und den Wert von 3 hat, bezogen auf die chemische Natur von Al, **dadurch gekennzeichnet, dass** es mittels eines Verfahrens erhalten wird, umfassend die nachstehenden aufeinanderfolgenden Schritte:
(i) Inkontaktbringen des genannten Titankomplexes (a) und Umsetzen, in einem geeigneten inerten flüssigen Medium, mit einer Menge der genannten Oxyhydrocarbyl-Verbindung von Aluminium (b), so dass das Atomverhältnis Al/Ti im Bereich von 50 bis 2000, vorzugsweise von 300 bis 1000, liegt, und
(ii) Inkontaktbringen des Produktes aus dem vorhergehenden Schritt (i) und Umsetzen mit der genannten organometallischen Hydrocarbylverbindung (c) in einer solchen Menge, dass das Atomverhältnis Al/Ti im Bereich von 20 bis 200 liegt.

2. Zusammensetzung nach Anspruch 1, wobei in der Formel (I) das genannte R ausgewählt ist aus Hydrid, Halid, einer C₁-C₈ Alkylgruppe, einer C₃-C₁₂ Alkylsilylgruppe, einer C₅-C₈ Cycloalkylgruppe, einer C₆-C₁₀ Arylgruppe, einer C₁-C₈ Alkoxylgruppe, einer C₁-C₃₀ Carboxylgruppe, einer C₂-C₁₀ Dialkylamidgruppe, und die Zahl "m" gleich 1 ist.

3. Zusammensetzung nach einem der Ansprüche 1 und 2, wobei die Gruppe A der Formel (I) unterschiedlich von einem η⁵-Cyclopentadienylring ist, umfassend einen Arylmethylsubstituent.

4. Zusammensetzung nach einem der vorhergehenden Ansprüche, wobei die genannte Verbindung der Formel (I) der Komponente (a) löslich in dem flüssigen Medium aus dem Schritt (i) ist.

5. Zusammensetzung nach einem der vorhergehenden Ansprüche, wobei die genannte Oxyhydrocarbyl-Verbindung von Aluminium in der Komponente (b) ein polymeres Aluminoxan ist, vorzugsweise ein Methylaluminoxan.

6. Zusammensetzung nach einem der vorhergehenden Ansprüche, wobei in dem genannten Schritt (ii) das Al/Al-Verhältnis zwischen dem Aluminium in der genannten Oxyhydrocarbyl-Verbindung (b) und dem Aluminium in (c) im Bereich von 1 bis 20 liegt.

7. Zusammensetzung nach einem der vorhergehenden Ansprüche, wobei die genannten Schritte (i) und (ii) in einem Reaktor für die Trimerisierung von Ethylen *in situ* durchgeführt werden.

8. Zusammensetzung nach einem der vorhergehenden Ansprüche, wobei das genannte inerte flüssige Medium aus aromatischen Kohlenwasserstoffen, vorzugsweise Toluol, ausgewählt ist.

9. Zusammensetzung nach einem der vorhergehenden Ansprüche, welche einer Alterungszeit nach Beenden des Schritts (i) unterzogen wird.

10. Zusammensetzung nach einem der vorhergehenden Ansprüche, welche einer Alterungszeit nach Beenden des Schritts (ii) unterzogen wird.

11. Verfahren zur Oligomerisierung von Ethylen zu vorherrschenden Herstellung von 1-Hexen, **dadurch gekennzeichnet, dass** Ethylen oder ein Ethylen enthaltendes Gas unter geeigneten Bedingungen mit der genannten katalytischen Zusammensetzung nach einem der vorhergehenden Ansprüche 1 bis 10 in Kontakt gebracht wird.

12. Verfahren nach Anspruch 11, wobei die genannte Oligomerisierung von Ethylen bei einer Temperatur im Bereich von 0°C bis 100°C und einem Druck niedriger als 10 MPa durchgeführt wird.

13. Verfahren nach Anspruch 12, wobei die genannte Oligomerisierung von Ethylen bei einer Temperatur im Bereich von 10°C bis 50°C und einem Druck im Bereich von 0,05 bis 7 MPa durchgeführt wird.

14. Verfahren nach Anspruch 12 oder 13, wobei die genannte Oligomerisierung in Gegenwart eines Lösungsmittels und/oder Verdünnungsmittels durchgeführt wird.

15. Verfahren nach Anspruch 14, wobei das genannte Lösungsmittel/Verdünnungsmittel aus aromatischen Kohlenwasserstoffen ausgewählt ist.

16. Verfahren nach einem der Ansprüche 11 bis 15, wobei die genannte katalytische Zusammensetzung vor dem Kontakt mit Ethylen vorgeformt wird.

17. Verfahren nach einem der Ansprüche 11 bis 15, wobei die genannte katalytische Zusammensetzung *in situ* in Gegenwart von Ethylen geformt wird.

18. Verfahren nach einem der Ansprüche 11 bis 17, umfassend mindestens einen Abtrennungsschritt von 1-Hexen.

## Revendications

1. Composition catalytique utilisable dans la trimérisation sélective d'éthylène en 1-hexène, comprenant le produit de la réaction de :
a) un composé monocyclopentadiényle de titane de formule (I) suivante :
[ATiRₙ]ₘ (I)
dans laquelle :
Ti est un atome de titane dans un état d'oxydation de +3 ou, de préférence, +4 ;
A est un anion contenant un cycle η⁵-cyclopentadiényle coordonné à l'atome de titane ;
chaque R représente indépendamment un groupe lié de manière covalente par liaison σ à l'atome de titane, choisi parmi un hydrure, un halogénure, un groupe alkyle en C₁ à C₈, un groupe alkylsilyle en C₃ à C₁₂, un groupe cycloalkyle en C₅ à C₈, un groupe aryle en C₆ à C₁₀, un groupe alcoxy en C₁ à C₈, un groupe carboxyle en C₁ à C₃₀, un groupe dialkylamide en C₂ à C₁₀;
"n" est égal à 2 ou 3 quand le titane est dans un état d'oxydation de +3 ou +4, respectivement ;
"m" est un entier de 1 à 4;
b) un composé oxyhydrocarbyle non monomère d'aluminium, et
c) un composé hydrocarbyle organométallique choisi parmi les composés de formule (III) suivante :
(R')ₘMXₛ₋ₘ (III)
dans laquelle
M est Al,
R' est un groupe hydrocarbyle ayant de 1 à 15 atomes de carbone,
X est le chlore ou le brome,
m est un entier de 1 à s, de préférence s,
s est la valence du métal Al et vaut 3, conformément à la nature chimique de l'Al,
**caractérisée en ce qu'**on l'obtient au moyen d'un procédé comprenant les étapes successives suivantes, consistant à :
i) mettre en contact ledit complexe de titane (a) et le faire réagir, dans un milieu liquide inerte convenable, avec une quantité dudit composé oxyhydrocarbyle d'aluminium (b), de façon que le rapport atomique Al/Ti soit situé dans la plage allant de 50 à 2000, de préférence de 300 à 1000, et
ii) mettre en contact le produit de l'étape (i) précédente et le faire réagir avec ledit composé hydrocarbyle organométallique (c) en une quantité telle que le rapport atomique Al/Ti soit situé dans la plage allant de 20 à 200.

2. Composition selon la revendication 1, dans laquelle, dans la formule (I), ledit R est choisi parmi un hydrure, un halogénure, un groupe alkyle en C₁ à C₈, un groupe alkylsilyle en C₃ à C₁₂, un groupe cycloalkyle en C₅ à C₈, un groupe aryle en C₆ à C₁₀, un groupe alcoxy en C₁ à C₈, un groupe carboxyle en C₁ à C₃₀, un groupe dialkylamide en C₂ à C₁₀, et ledit indice "m" est égal à 1.

3. Composition selon l'une quelconque des revendications 1 et 2, dans laquelle le groupe A dans la formule (I) est différent d'un cycle η⁵-cyclopentadiényle comprenant un substituant arylméthyle.

4. Composition selon l'une quelconque des revendications précédentes, dans laquelle ledit composé de formule (I) du composant (a) est soluble dans le milieu liquide de l'étape (i).

5. Composition selon l'une quelconque des revendications précédentes, dans laquelle ledit composé oxyhydrocarbyle d'aluminium dans le composant (b) est un aluminoxane polymère, de préférence le méthylaluminoxane.

6. Composition selon l'une quelconque des revendications précédentes, dans laquelle, dans ladite étape (ii), le rapport Al/Al entre l'aluminium dans ledit composé oxyhydrocarbyle (b) et l'aluminium dans (c) est situé dans la plage allant de 1 à 20.

7. Composition selon l'une quelconque des revendications précédentes, dans laquelle lesdites étapes (i) et (ii) sont effectuées *in situ* dans un réacteur pour la trimérisation d'éthylène.

8. Composition selon l'une quelconque des revendications précédentes, dans laquelle ledit milieu liquide inerte est choisi parmi les hydrocarbures aromatiques, de préférence le toluène.

9. Composition selon l'une quelconque des revendications précédentes, soumise à une période de vieillissement après l'achèvement de l'étape (i).

10. Composition selon l'une quelconque des revendications précédentes, soumise à une période de vieillissement après l'achèvement de l'étape (ii).

11. Procédé pour l'oligomérisation d'éthylène afin de produire principalement du 1-hexène, **caractérisé en ce que** de l'éthylène, ou un gaz contenant de l'éthylène, est mis en contact, dans des conditions convenables, avec ladite composition catalytique selon l'une quelconque des revendications 1 à 10 précédentes.

12. Procédé selon la revendication 11, dans lequel ladite oligomérisation d'éthylène est effectuée à une température située dans la plage allant de 0°C à 100°C et sous une pression inférieure à 10 MPa.

13. Procédé selon la revendication 12, dans lequel ladite oligomérisation d'éthylène est effectuée à une température située dans la plage allant de 10°C à 50°C et sous une pression située dans la plage allant de 0,05 à 7 MPa.

14. Procédé selon la revendication 12 ou 13, dans lequel ladite oligomérisation est effectuée en présence d'un solvant et/ou d'un diluant.

15. Procédé selon la revendication 14, dans lequel ledit solvant/diluant est choisi parmi les hydrocarbures aromatiques.

16. Procédé selon l'une quelconque des revendications 11 à 15, dans lequel ladite composition catalytique est formée au préalable avant contact avec l'éthylène.

17. Procédé selon l'une quelconque des revendications 11 à 15, dans lequel ladite composition catalytique est formée in situ, en présence d'éthylène.

18. Procédé selon l'une quelconque des revendications 11 à 17, comprenant au moins une étape de séparation de 1-hexène.
